# EUROPEAN PATENT APPLICATION

(11) **EP 4 831 299 A1**
(43) Date of publication of application: **28.10.2026**
(21) Application number: 24906432.0
(22) Date of filing: 19.12.2024
(51) Int. Cl.: C12Q 1/6883, A61K 31/192, A61P 27/02

(54) **VIDEO DISPLAY TERMINAL ASSOCIATED DRY EYE DISEASE DIAGNOSTIC BIOMARKER BASED ON EPITHELIAL-MESENCHYMAL TRANSITION AND FIBROSIS, AND THERAPEUTIC DRUG**

(30) Priority: 19.12.2023 CN 202311752725
(71) Applicant: The Eye Hospital of Wenzhou Medical University, Wenzhou, Zhejiang 325000 (CN)
(72) Inventor: CHEN, Wei, Wenzhou, Zhejiang 325027 (CN); LIU, Zihao, Wenzhou, Zhejiang 325027 (CN); XIE, He, Wenzhou, Zhejiang 325027 (CN)
(74) Representative: Gille Hrabal Partnerschaftsgesellschaft mbB Patentanwälte
(86) International application number: PCT/CN2024/140495
(87) International publication number: WO 2025/130953

(57) **Abstract**

Provided are a video display terminal (VDT) associated dry eye disease diagnostic biomarker based on epithelial-mesenchymal transition and fibrosis, and a therapeutic drug. By studying the functional changes of epithelial cells in VDT-associfated dry eye disease, the epithelial-mesenchymal transition of ocular surface epithelial cells in VDT-associated dry eye disease is disclosed, and in the acute phase of the disease course, the epithelial cells show fibroblast characteristics and pro-inflammatory characteristics, such that the acute phase and the chronic phase of VDT-associated dry eye disease are distinguished. Provided is a biomarker for diagnosing VDT-associated dry eye disease or monitoring treatment efficacy, and a reagent or kit that is prepared on the basis of the biomarker and used for diagnosing VDT-associated dry eye disease, monitoring the treatment efficacy, or distinguishing the acute and chronic courses of patients with VDT-associated dry eye disease . In addition, intervention targeting the epithelial-mesenchymal transition and fibrosis processes is an effective strategy for relieving VDT-associated dry eye disease and improving ocular surface damage, which provides potential intervention directions and prospects for clinical treatment of VDT-associated dry eye disease.

## Description

### Cross-Reference to Related Application

The present disclosure claims priority to the prior application filed with the China National Intellectual Property Administration on December 19, 2023, with application number 202311752725.1, titled "Diagnostic Biomarker for Dry Eye Disease Based on Epithelial-Mesenchymal Transition and Fibrosis and Use Thereof". The entire content of the prior application is incorporated by reference into the present disclosure.

### Technical Field

The present disclosure relates to the fields of biomarkers and medicine, and in particular to a biomarker for video display terminal-associated dry eye disease and a therapeutic agent for the dry eye disease.

### Background Art

Dry eye disease (DED) is a multifactorial chronic ocular surface disease and is one of the most common ophthalmic diseases in ophthalmology clinics. Most patients with dry eye disease have a chronic course, with symptoms presenting in an intermittent rather than persistent pattern. It is widely believed that most patients with chronic dry eye disease experience acute episodes, and the acute phase, chronic phase, and acute exacerbation in chronic phase of dry eye disease involve different immunological mechanisms. With the popularization of video display terminal (VDT) and changes in modern lifestyles, VDT-associated dry eye disease has become the most predominant type of dry eye disease in recent years, with its incidence increasing year by year and showing a trend of younger patient populations. Unlike other types of dry eye disease, VDT-associated dry eye disease is primarily caused by adverse visual environmental factors (such as prolonged staring at electronic screens, reduced blink frequency, and being in air-conditioned environments), rather than by systemic factors. It has been clearly established that long-term use of video display terminals is one of the independent risk factors for the development and progression of dry eye disease.

The main symptoms of dry eye disease in a patient include ocular discomfort (such as dryness, stinging, tearing, and eye fatigue) and visual impairment. These symptoms can severely affect the patient's quality of life and daily activities (including reading, computer use, and driving), and are also highly correlated with anxiety and depression. In addition to the indirect economic losses due to reduced work efficiency, the long-term treatment of dry eye disease also imposes a huge direct economic burden, and dry eye disease has become a public health concern of significant social importance. Current diagnosis of dry eye disease is usually based on subjective symptoms, tear break-up time (evaluating tear film quality), vital dye staining of the ocular surface (e.g., corneal fluorescein staining), Schirmer test (evaluating tear quality), and other less common clinical tests including tear osmolarity, Rose Bengal staining, measurement of tear meniscus height, etc. However, poor precision in clinical measurements of DED, the lack of a "gold standard" for determining disease severity, the inconsistency between patient-reported ocular symptoms and clinical signs as well as between different clinical parameters, and significant patient heterogeneity have been major challenges in clinical diagnosis of DED and clinical development of therapeutic interventions.

Findings elucidating the fundamental inflammatory mechanisms and pathways in DED have promoted a deeper understanding of the role of inflammation in the pathogenesis of this disease. Ocular surface epithelial cells play a key role in the development and progression of inflammatory responses in dry eye disease. HLA-DR level is elevated in the conjunctival epithelium of patients with dry eye disease , which has been used as a biomarker in clinical trials. In addition, epithelial dysfunction induced by dry environments can trigger innate immune responses and subsequent adaptive immune responses, further supporting the critical role of epithelial activation in dry eye disease. However, current research still has many gaps in the regulation of the immune microenvironment and immune responses involving epithelial cells. The regulation of the pro-inflammatory effects of epithelial cells and their interaction patterns with immune cells remain understudied, and further research is needed to identify key targets for the development and chronic persistence of dry eye inflammation, thereby enabling more effective diagnosis and treatment of dry eye disease. Furthermore, the acute and chronic courses of dry eye disease involve different pathogenic mechanisms: acute dry eye disease is associated with the release of inflammatory factors from the ocular surface epithelium stimulated by a hyperosmolar tear environment, while chronic dry eye disease may be associated with the dysfunction of ocular surface immune homeostasis. This emphasizes the importance of targeted therapy, and understanding these differences is crucial for developing effective therapeutic strategies and improving treatment outcomes. Particularly for the subtype of VDT-associated dry eye disease, there is an urgent need to clarify its specific pathogenesis to develop specialized diagnostic and therapeutic approaches.

### Summary

To address the problems existing in the prior art, through in-depth investigation of the functional changes of epithelial cells in VDT-associated dry eye disease, the present disclosure reveals for the first time the occurrence of epithelial-mesenchymal transition (EMT) in ocular surface epithelial cells in VDT-associated dry eye disease, and for the first time elucidates that epithelial cells exhibit fibroblast characteristics and pro-inflammatory properties during the acute phase of dry eye disease, thereby distinguishing the acute phase from the chronic phase of VDT-associated dry eye disease. Based on epithelial-mesenchymal transition and fibrosis indicators, more precise and effective diagnostic approaches for dry eye disease and methods for distinguishing between acute and chronic courses of VDT-associated dry eye disease in patients are provided. In addition, intervention targeting the epithelial-mesenchymal transition and fibrosis process is an effective strategy for alleviating VDT associated dry eye inflammation and improving ocular surface damage, thereby providing potential intervention directions and prospects for clinical treatment of VDT-associated dry eye disease.

The primary objective of the present disclosure is to provide a biomarker for diagnosing a VDT-associated dry eye disease or monitoring efficacy of treatment for the VDT-associated dry eye disease, and use thereof. The second objective of the present disclosure is to provide a biomarker for distinguishing between acute and chronic courses of a VDT-associated dry eye disease in a patient. The third objective of the present disclosure is to provide use of an anti-fibrotic agent in the manufacture of a medicament for inhibiting epithelial-mesenchymal transition and fibrosis of ocular surface epithelial cells in a VDT-associated dry eye disease.

To achieve the objectives of the present disclosure, the technical solutions are as follows:
In a first aspect, the present disclosure provides use of a gene as a biomarker in VDT-associated dry eye disease in the manufacture of a reagent or kit for diagnosing VDT-associated dry eye disease or monitoring efficacy of treatment for the VDT-associated dry eye disease, wherein the gene comprises one or more selected from *AQP3, SNAI1, TWIST1, CCL2, CCL7, CCL8, IGFBP2, TGF-β,* and *MUC5AC*;
preferably, the genes comprise *TWIST1, SNAI1,* and *IGFBP2*.

In a second aspect, the present disclosure provides use of an agent for detecting the expression of a target gene in the manufacture of a reagent or kit for diagnosing VDT-associated dry eye disease or monitoring efficacy of treatment for VDT-associated dry eye disease, wherein the target gene comprises one or more of *AQP3, SNAI1, TWIST1, CCL2, CCL7, CCL8, IGFBP2, TGF-β,* and *MUC5AC*;
preferably, the genes comprise *TWIST1, SNAI1,* and *IGFBP2*.

In a third aspect, the present disclosure provides a reagent or kit for diagnosing VDT-associated dry eye disease or monitoring efficacy of treatment for VDT-associated dry eye disease, wherein the reagent comprises an agent for detecting the expression level of a gene in a biological test sample from a subject, wherein the gene is at least one of *AQP3, SNAI1, TWIST1, CCL2, CCL7, CCL8, IGFBP2, TGF-β,* and *MUC5AC*;
the kit comprises the agent; and
the biological test sample from the subject is obtained from conjunctival superficial cells of the ocular surface;
preferably, the genes comprise *TWIST1, SNAI1,* and *IGFBP2*.

In a fourth aspect, the present disclosure provides use of a gene as a biomarker in VDT-associated dry eye disease in the manufacture of a reagent or kit for distinguishing between acute and chronic courses of VDT-associated dry eye disease in a patient, wherein the gene is selected from one or more of *AQP3, CCL2, CCL7,* and *CCL8*.

In a fifth aspect, the present disclosure provides use of an agent for detecting the expression of a target gene in the manufacture of a reagent or kit for distinguishing between acute and chronic courses of VDT-associated dry eye disease in a patient, wherein the target gene comprises at least one of *AQP3, CCL2, CCL7, CCL8,* and *MUC5AC*;
preferably, the genes comprise *AQP3, CCL2,* and *MUC5AC*.

In a sixth aspect, the present disclosure provides a reagent or kit for distinguishing between acute and chronic courses of VDT-associated dry eye disease in a patient, wherein
the reagent comprises an agent for detecting the expression level of a gene in a biological test sample from a subject in which the gene is at least one of *AQP3, CCL2, CCL7, CCL8,* and *MUC5AC*;
preferably, the genes comprise *AQP3, CCL2,* and *MUC5AC*;
the kit comprises the agent; and
the biological test sample from the subject is obtained from conjunctival superficial cells of the ocular surface.

In a seventh aspect, the present disclosure provides a system for diagnosing VDT-associated dry eye disease, the system performing the following steps:
(1) detecting the expression level value of a corresponding gene in a biological test sample from a subject with the reagent according to the third aspect; and
(2) comparing the detected expression level value of the gene with the normal or reference expression level value of the gene.

Preferably, the biological test sample is obtained from conjunctival superficial cells of the ocular surface.

Further, the system for diagnosing VDT-associated dry eye disease or monitoring efficacy of treatment for VDT-associated dry eye disease comprises a data input module, a data comparison module, and a conclusion output module, wherein
the data input module is configured to input the detected expression level value of the target gene in the biological test sample from the subject;
the data comparison module is configured for comparing the expression level value of the target gene in the biological test sample from the subject with a control value, wherein the control value is the expression level value of the target gene in a sample from a healthy subject;
the conclusion output module is configured for outputting a conclusion according to the following criteria: if the expression level value of the target gene in the biological test sample from the subject is greater than or less than the control value, the subject has, or is suspected of having, VDT-associated dry eye disease;
the target gene comprise one or more of *SNAI1, TWIST1, IGFBP2,* and *MUC5AC*;
"greater than or less than" specifically can be a statistically significant "greater than or less than", wherein the target genes whose expression level values in the biological test sample from the subject are greater than the control value are *TWIST1, SNAI1,* and *IGFBP2*; and the target gene whose expression level value in the biological test sample from the subject is less than the control value is *MUC5AC*.

In an eighth aspect, the present disclosure provides use of an anti-fibrotic agent in the manufacture of a medicament for treating dry eye disease.

Preferably, the dry eye disease is a video display terminal associated dry eye disease, and the anti-fibrotic agent of the present disclosure is pirfenidone and a derivative thereof or tranilast and a derivative thereof.

Compared with the prior art, the beneficial effects of the present disclosure are as follows.

The present disclosure for the first time elucidates the occurrence of epithelial-mesenchymal transition (EMT) in ocular surface epithelial cells in VDT-associated dry eye disease, and that epithelial cells exhibit fibroblast characteristics and pro-inflammatory properties during the acute phase of VDT-associated dry eye disease, thereby distinguishing the acute phase from the chronic phase of VDT-associated dry eye disease. Utilizing single-cell sequencing and related studies, the present disclosure for the first time reveals biomarkers for diagnosing VDT-associated dry eye disease or monitoring efficacy of treatment for VDT-associated dry eye disease. With these biomarkers, a reagent or kit can be prepared for diagnosing VDT-associated dry eye disease, monitoring efficacy of treatment for VDT-associated dry eye disease, or distinguishing between acute and chronic courses of VDT-associated dry eye disease in a patient.

The biomarkers provided by the present disclosure contribute to a better understanding of the pathophysiology of VDT-associated dry eye disease, will provide new opportunities for diagnosis and prognosis, thereby improving clinical services for patients with VDT-associated dry eye disease.

In the present disclosure, pirfenidone or tranilast,as an anti-fibrotic agent is used to inhibit the epithelial-mesenchymal transition and fibrosis process starting from the baseline of a mouse VDT-associated dry eye disease model, which has significant effects on alleviating VDT-associated dry eye disease symptoms and reducing ocular surface damage.

### Brief Description of the Drawings

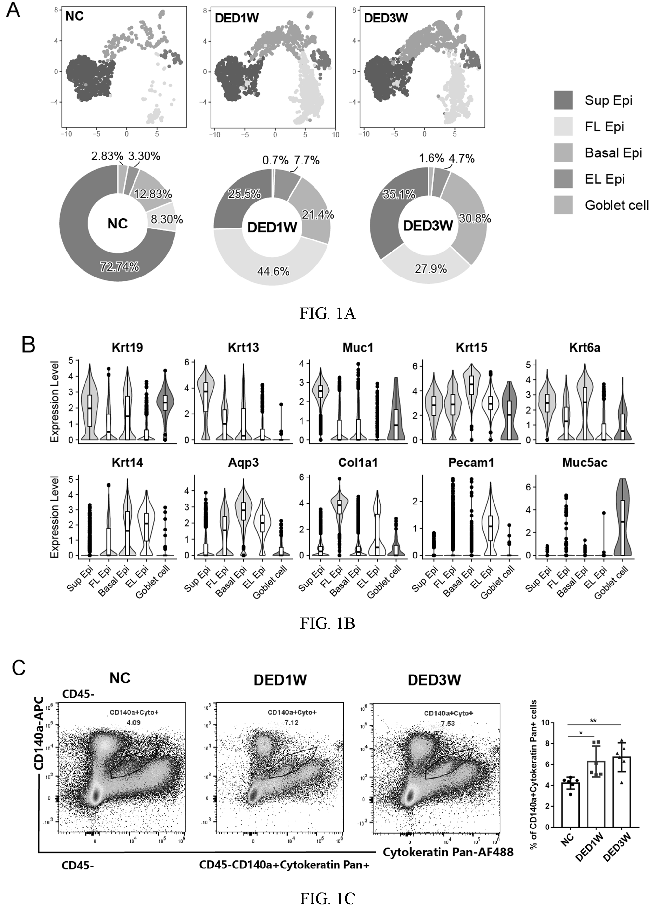

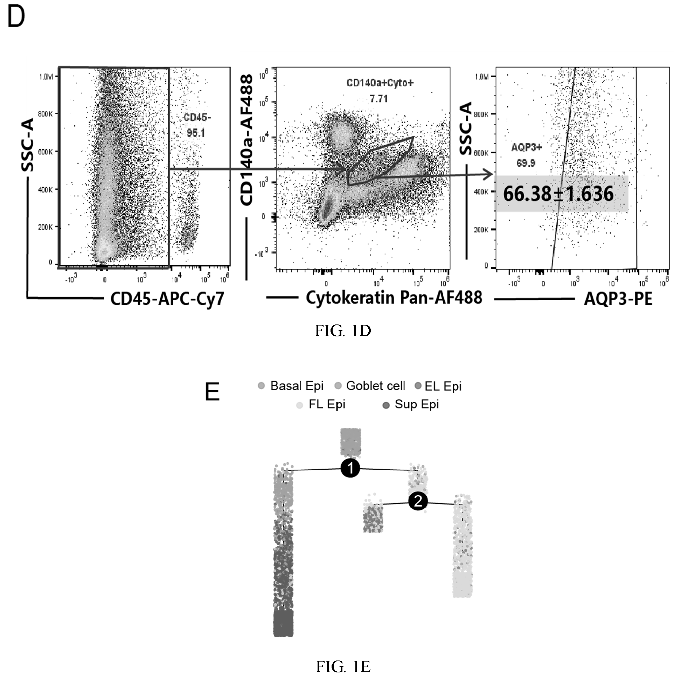

FIG. 1: A. Uniform Manifold Approximation and Projection (UMAP) plots of epithelial cell subpopulations in the normal group (NC) and ICES dry eye disease model groups at 1 week (DED1W) and 3 weeks (DED3W). Points are colored according to major cell types. B. Violin plots showing the expression of cell-specific marker genes used to identify epithelial cell subpopulation identities. C. Flow cytometry analysis of cell populations in mouse conjunctiva co-expressing the basal epithelial cell marker Cytokeratin Pan and the fibroblast marker CD140a, and their proportion among non-immune cells. D. Flow cytometry analysis of the proportion of AQP3-positive fibroblast-like epithelial cells in the conjunctiva of dry eye disease model mice. E. Cell trajectory analysis results by Monocle2, colored by cell subpopulations. Numbers 1 and 2 indicate potential differentiation nodes between cell subpopulations.

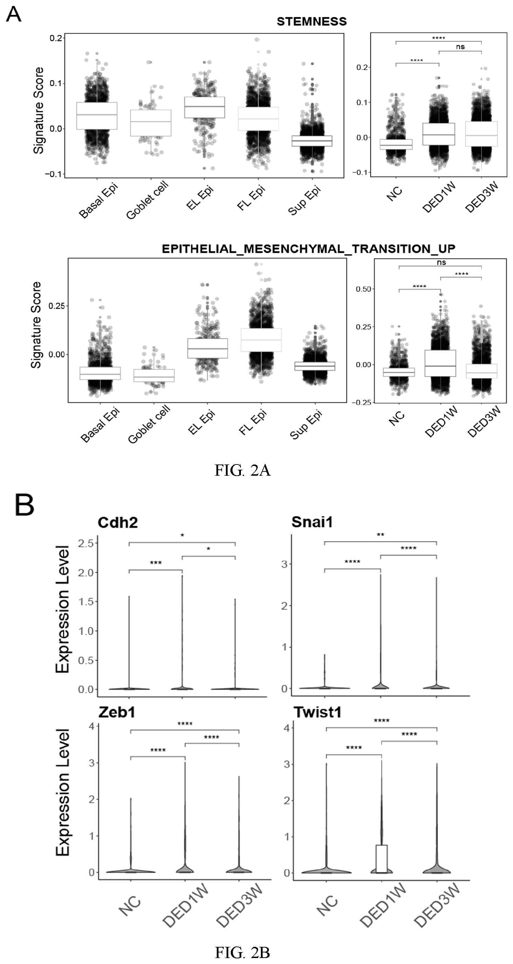

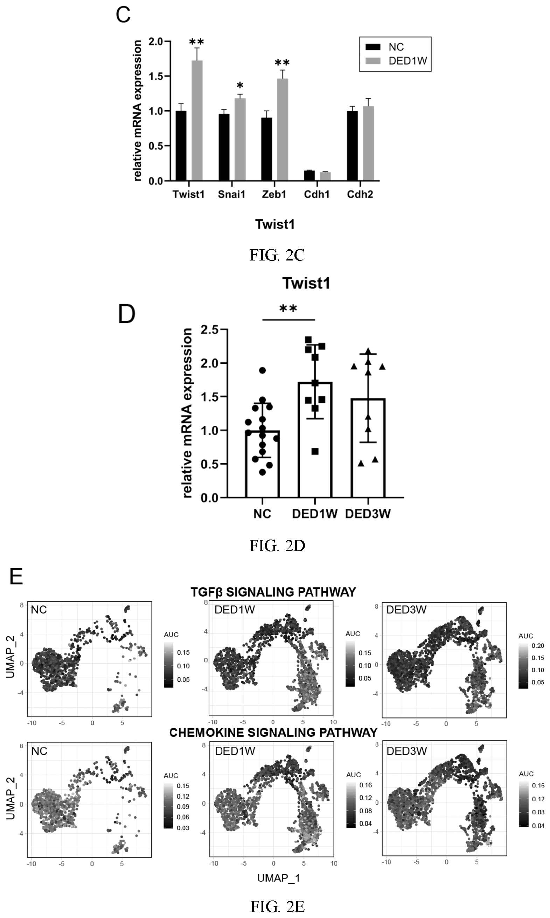

FIG. 2: A. Gene set signature score of epithelial cell subpopulations (left) and different stages of dry eye disease (right) using stemness gene sets and epithelial-mesenchymal transition gene sets. P values are calculated by two-sided Wilcoxon rank-sum test. B. Violin plots showing the expression levels of epithelial-mesenchymal transition markers (CDH2, SNAI1, ZEB1, TWIST1) in epithelial cells at different stages of dry eye disease. P values are calculated by two-sided Wilcoxon rank-sum test. C. Real-time quantitative PCR evaluating the expression levels of epithelial-mesenchymal transition markers (Twist1, Snai1, Zeb1, Cdh1, Cdh2) in the conjunctiva at different stages of dry eye disease. n=9, mean ± SEM, P values are calculated by unpaired two-sided t-test. D. Real-time quantitative PCR evaluating the expression level of TWIST1 in the conjunctiva at different stages of dry eye disease. Mean±SEM, P values are calculated by one-way ANOVA. E. UMAP plots colored by TGF-β signaling pathway and chemokine signaling pathway activity.

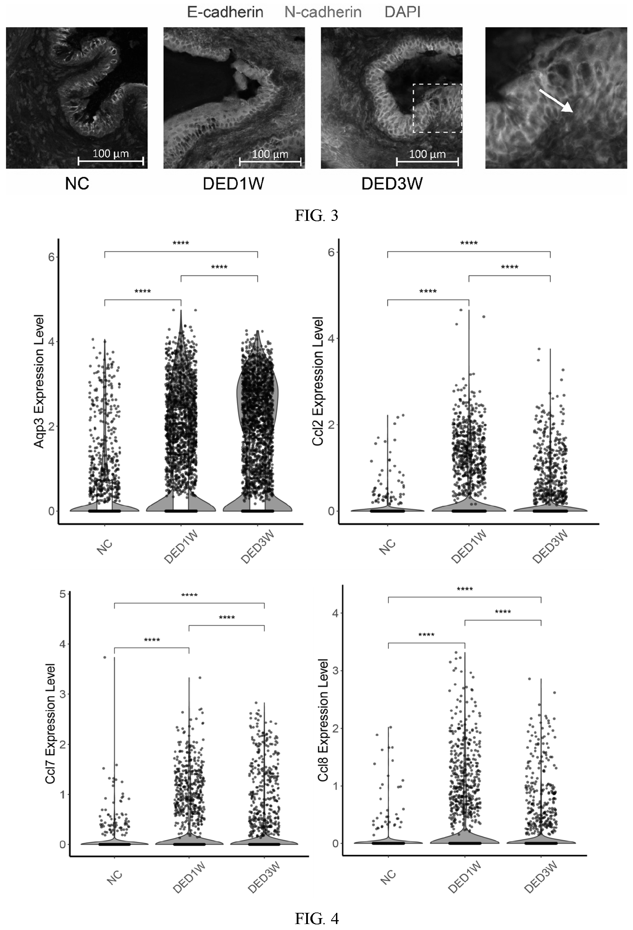

FIG. 3: Representative immunofluorescence images from immunofluorescence analysis of conjunctival tissue frozen sections at three stages of dry eye disease: E-cadherin (red), N-cadherin (green), and DAPI (blue). Arrows indicate migration of conjunctival epithelial cells toward the stroma.

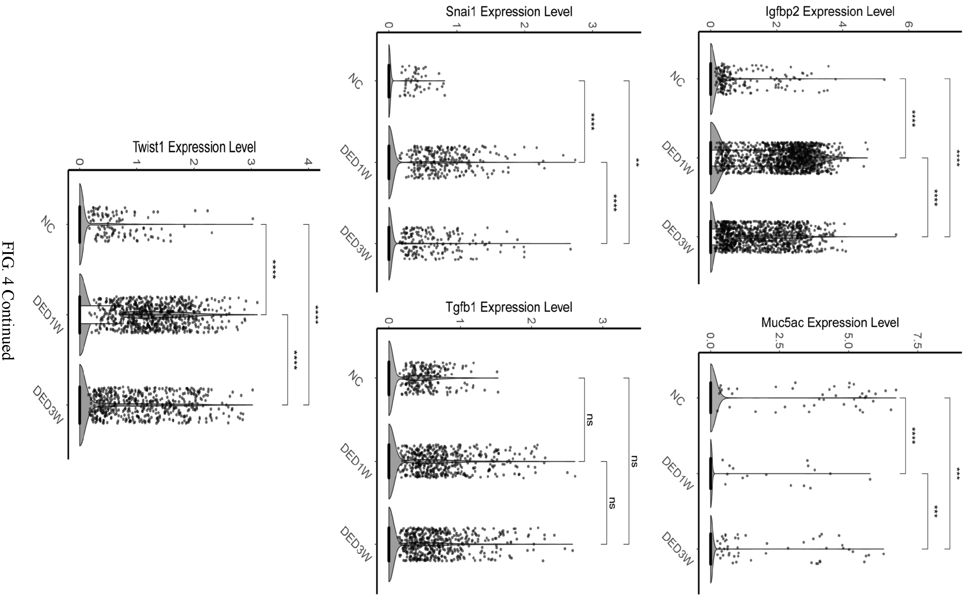

FIG. 4: Violin plots showing the expression levels of key biomarkers in epithelial cells at different stages of dry eye disease. P values are calculated by two-sided Wilcoxon rank-sum test.

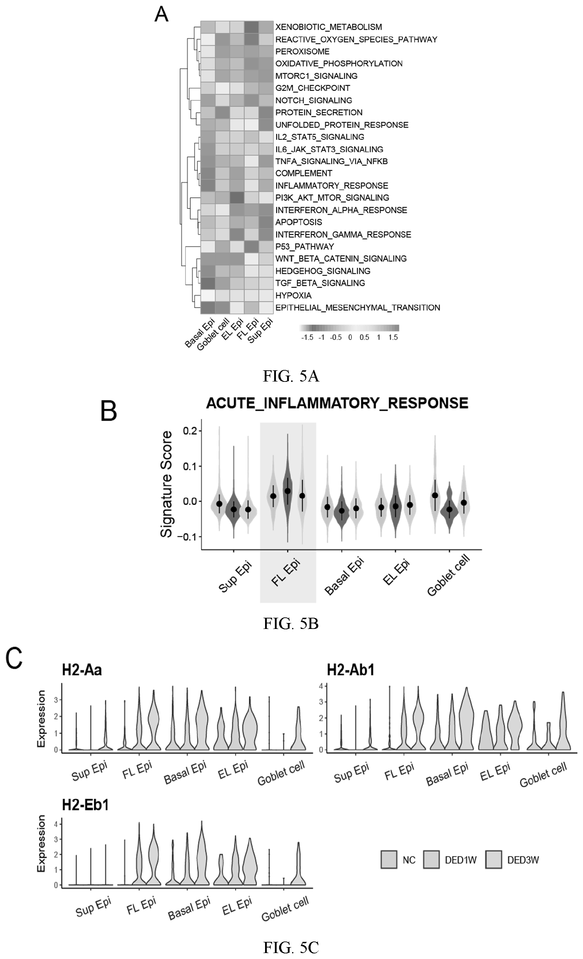

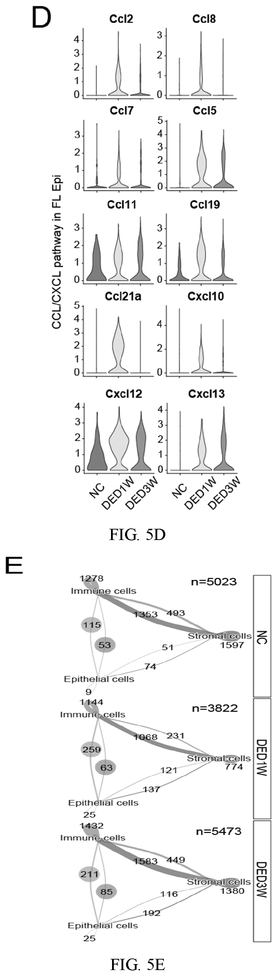

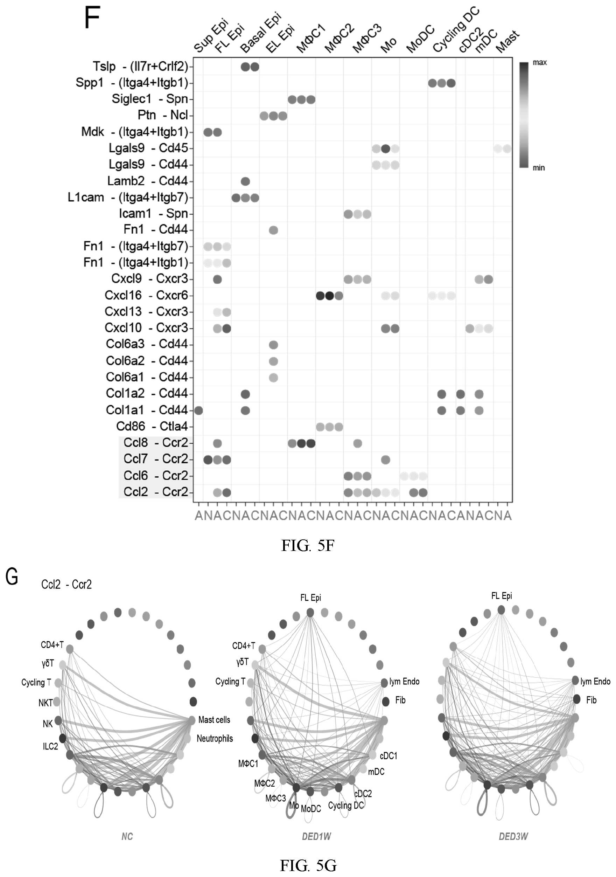

FIG. 5: A. Evaluation of pathway activity differences among epithelial cell subpopulations in dry eye disease using GSVA based on Hallmark gene sets. B. Acute inflammatory response gene set signature scores of epithelial cell subpopulations during the course of dry eye disease. Each subpopulation shows scores from left to right for normal, dry eye disease 1 week, and dry eye disease 3 weeks. Gene set is from GO database (GO:0002526). C. Violin plots showing the expression of MHC-II molecule genes in epithelial cell subtypes at different stages of dry eye disease. D. Violin plots showing the expression of representative genes within the CCL/CXCL signaling pathway in fibroblast-like epithelial cells. E. Total number of cell-cell interactions among immune cells, epithelial cells, and stromal cells. F. Dot plot showing ligand-receptor pairs upregulated in dry eye disease 1W with CD4+T cells as signal receivers. Epithelial cells and myeloid cell subpopulations are signal senders. All signals have P values < 0.01. G. CCL2-CCR2 signaling network during the course of dry eye disease. Nodes represent major cell types, and line thickness indicates interaction strength.

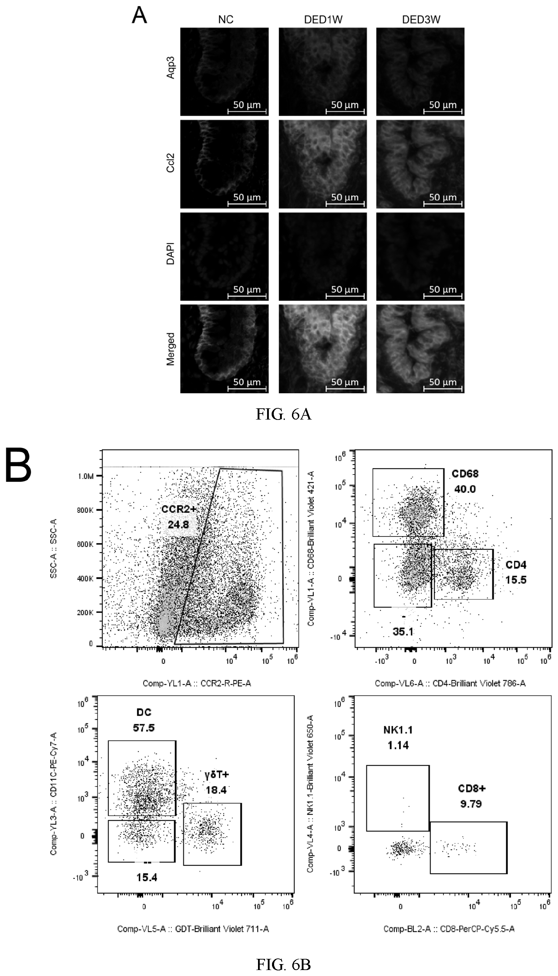

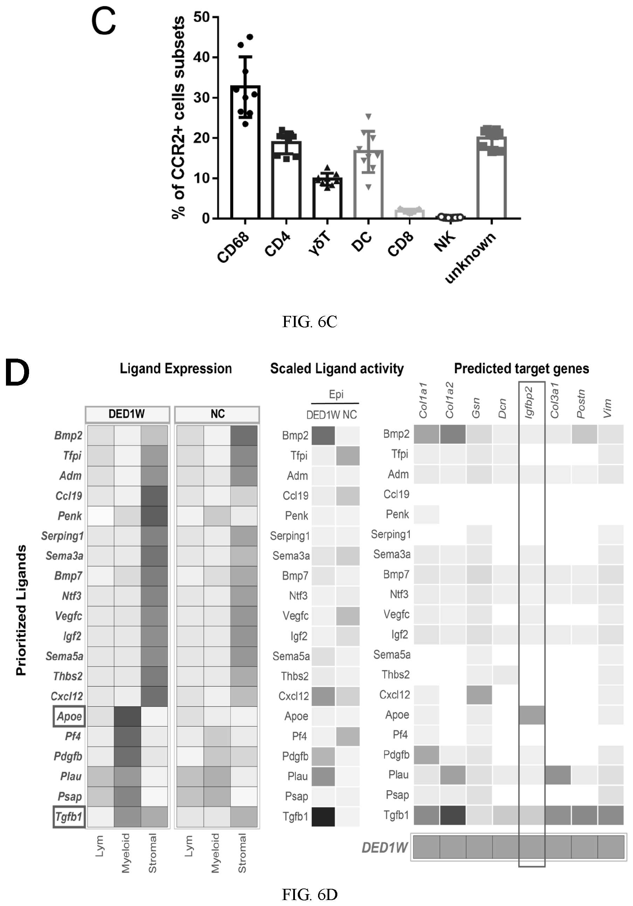

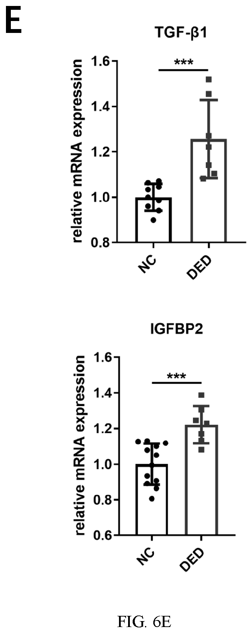

FIG. 6: A. Immunofluorescence detection of conjunctival tissue sections from dry eye disease mice using anti-AQP3 (marking basal epithelial cells, red) and anti-CCL2 (green) antibodies. B and C. Flow cytometry analysis of CCR2-expressing cell populations in mouse conjunctiva, showing population distribution diagram (B) and proportions of each cell type (C). D. NicheNet-based ligand-target gene prediction. Heatmap shows the expression of ligands sent by lymphocytes, myeloid cells, and stromal cells in dry eye disease 1W and normal groups, and the corresponding target gene expression in epithelial cells. E. Real-time quantitative PCR evaluating the expression levels of TGF-β1 and IGFBP2 in the conjunctiva of dry eye disease mice. Mean±SEM, P values are calculated by unpaired two-sided t-test.

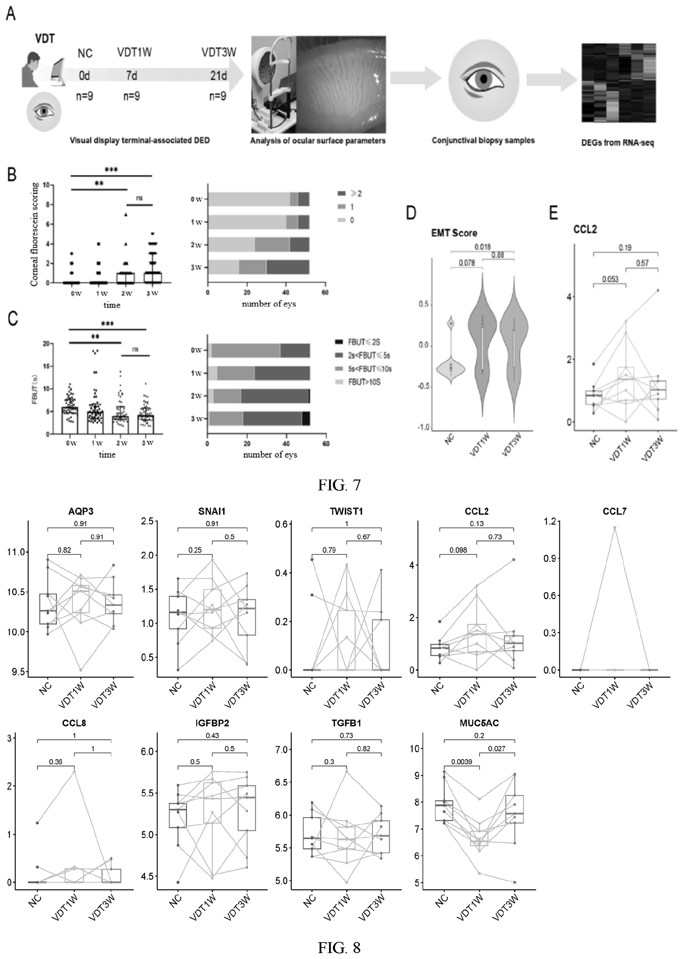

FIG. 7: A. Schematic diagram of the experimental design for the effect of prolonged VDT use on the ocular surface microenvironment. B. Corneal fluorescein sodium scores and the number of eyes by score grade at each time point. C. Tear break-up time and the number of eyes by score grade at each time point. D. EMT signature scores of the three groups calculated based on GSVA. P values are calculated by paired t-test. EMT gene set is from GO database (GO:0010718). E. Expression levels of CCL2 in the three groups.
FIG. 8: Changes in expression levels of key biomarkers in the ocular surface microenvironment at various time points of prolonged VDT use. P values are calculated by paired t-test.

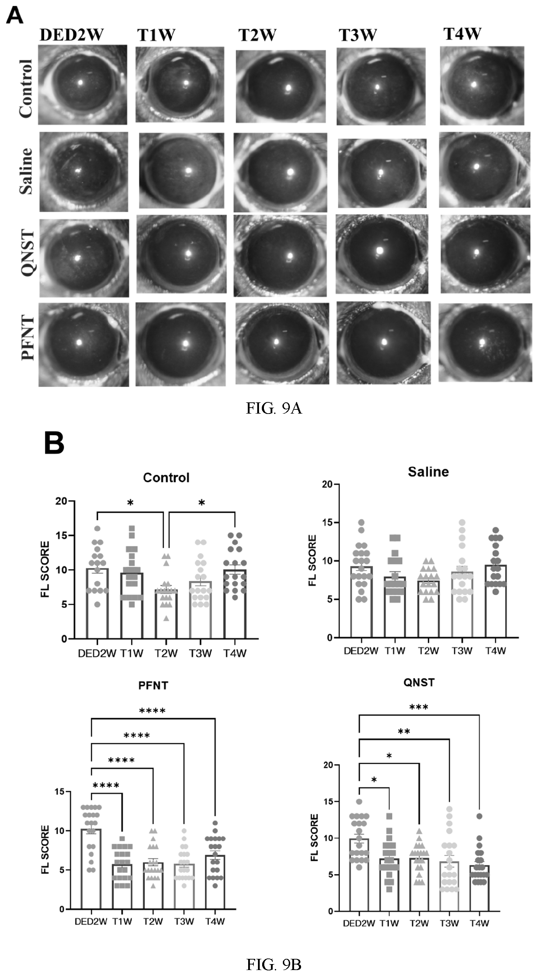

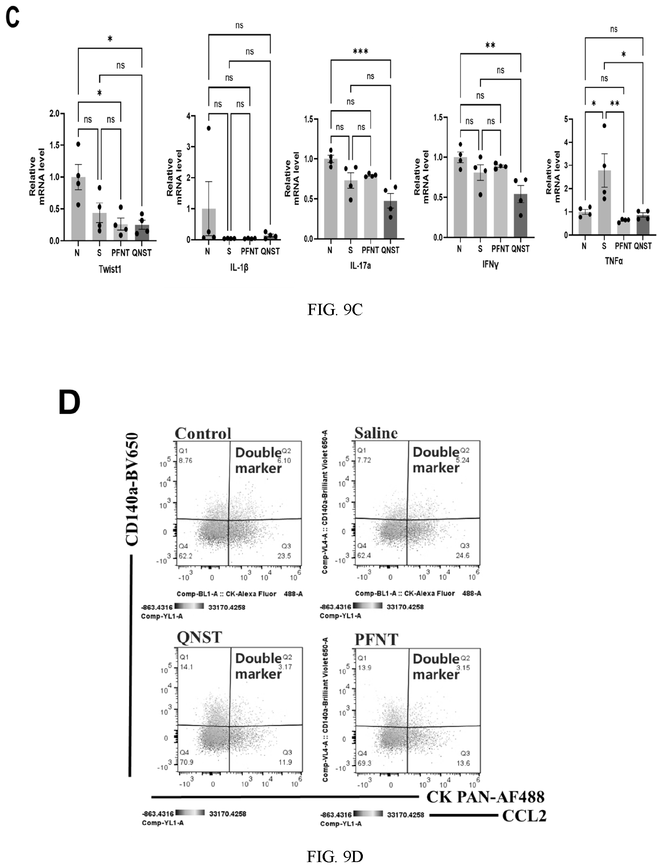

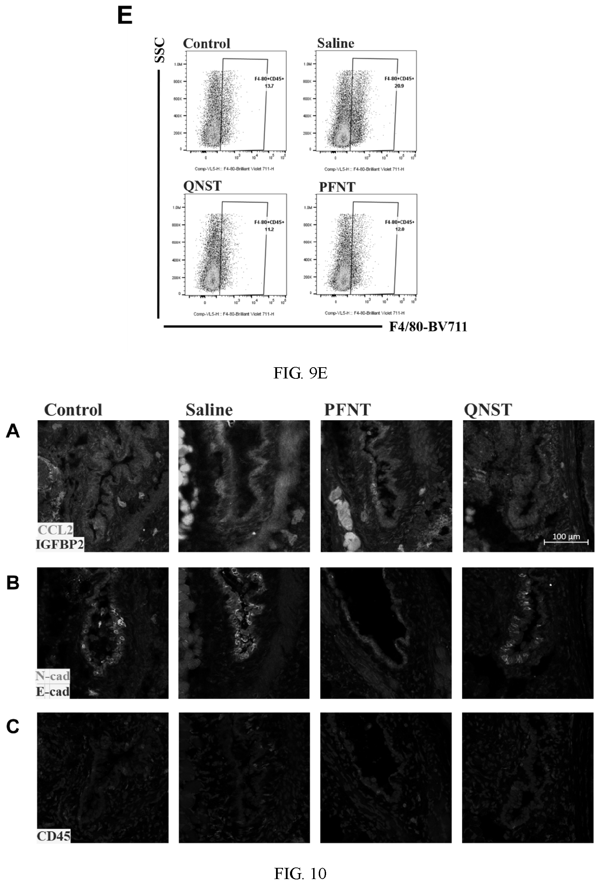

FIG. 9: Results of multi-level analysis before and after treatment of the VDT-associated dry eye disease model.
   A and B. Representative corneal fluorescein sodium staining images (A) and quantitative analysis of staining scores (B) of mice in the untreated dry eye disease model group (Control), dry eye disease + normal saline eye drop group, dry eye disease + pirfenidone eye drop group, and dry eye disease + tranilast eye drop group at different time points after drug administration observed under a slit-lamp microscope. * P<0.05, ** P<0.01, *** P<0.001.
   C. The relative mRNA expression levels of the epithelial-mesenchymal transition (EMT) key transcription factor Twist1 and inflammatory factors IL-1β, IL-17a, IFN-γ, and TNF-α in the conjunctival epithelium of mice in each group detected by real-time fluorescence quantitative PCR. Data are presented as Mean±SEM and analyzed by one-way ANOVA.
   D. Flow cytometry analysis of the double-positive cell population co-expressing the epithelial cell marker CK PAN and fibroblast marker CD140a in the conjunctival tissue of mice in each group, and their CCL2 expression levels.
   E. Flow cytometry analysis of the proportion of F4/80-positive macrophages among CD45-positive immune cell populations in the conjunctival tissue of mice in each group.
FIG. 10: Immunofluorescence detection results of epithelial-mesenchymal transition and fibrosis associated indicators in the conjunctiva before and after treatment of the VDT-associated dry eye disease model;
   A. Anti-CCL2 (green) and anti-IGFBP2 (red) antibody staining shows that after treatment with the two anti-fibrotic agents, the expression of CCL2 and IGFBP2 in conjunctival tissue is significantly reduced; scale bar: 100 µm;
   B. Anti-N-cadherin (green) and anti-E-cadherin (red) antibody staining shows that after anti-fibrotic agent treatment, the expression of the epithelial mesenchymal transition marker N-cadherin induced by dry eye disease is downregulated; scale bar: 100 µm;
   C. Anti-CD45 (red) antibody staining shows that immune cell infiltration in the conjunctival stroma is significantly increased in the dry eye disease model group, and is significantly reduced after anti-fibrotic agent treatment; scale bar: 100 µm; nuclei are stained with DAPI (blue).

### Detailed Description

### Definitions

Unless otherwise defined or clearly indicated by the context, all technical and scientific terms used in this disclosure have the same meanings as commonly understood by one having ordinary skill in the art to which this disclosure belongs.

Unless otherwise stated, the terms "comprising" and "including" are used herein in their open-ended and non-limiting sense. It should be further understood that where the description of various embodiments uses the terms "comprising" or "including", those skilled in the art will appreciate that in some specific instances, the examples may alternatively be described using the language "consisting essentially of" or "consisting of".

Although the disclosed content supports the definition of the term "or" as merely an alternative as well as "and/or", the term "or" in the claims refers to "and/or" unless expressly indicated to be merely an alternative or mutually exclusive among alternatives.

In the present disclosure, the term "dry eye disease (DED)" refers to a multifactorial chronic ocular surface disease caused by abnormalities in the quality, quantity, and dynamics of tears leading to tear film instability or imbalance of the ocular surface microenvironment, which may be accompanied by ocular surface inflammatory reactions, tissue damage, and neurological abnormalities, and can cause various ocular discomfort symptoms and/or visual dysfunction.

In the present disclosure, "video display terminal (VDT)" refers to a device for visual display that can present information stored and electronically processed in the form of symbols, graphics, or a combination of both.

In the present disclosure, the term "VDT-associated dry eye disease" specifically refers to a chronic ocular surface disease caused by lifestyle factors such as prolonged use of video display terminals, leading to abnormal tear dynamics (such as reduced blink frequency, incomplete blinks, etc.) and increased tear evaporation, resulting in tear film instability or imbalance of the ocular surface microenvironment, which may be accompanied by ocular surface inflammatory reactions and tissue damage, causing ocular discomfort symptoms and visual dysfunction. VDT-associated dry eye disease is one of the major subtypes of dry eye disease, and its pathogenesis is closely related to prolonged exposure to VDT environments.

In the present disclosure, the term "biomarker" refers to a biochemical indicator that can mark changes or possible changes in the structure or function of systems, organs, tissues, cells, and subcellular structures. It can be used for disease diagnosis, determining disease stage, or evaluating the safety and efficacy of new drugs or new therapies in target populations.

In the present disclosure, "epithelial-mesenchymal transition (EMT)" refers to the process by which epithelial cells lose cell-cell adhesion and polarity, gain migratory and invasive capabilities, and thereby transform into mesenchymal cells, playing important roles in embryonic development, chronic inflammation, tissue remodeling, cancer metastasis, and various fibrotic diseases.

In the present disclosure, "conjunctival fibrosis" refers to a pathological process in which the conjunctival tissue of the eye undergoes excessive collagen deposition due to tissue damage, chronic inflammation, or post-surgical reactions, leading to the replacement of normal conjunctival tissue by fibrous tissue, thereby impairing conjunctival function.

In the present disclosure, "acute course of VDT-associated dry eye disease" refers to the initial stage of VDT-associated dry eye disease onset, usually caused by stimulation or stress to the ocular surface. In this stage, the innate immune system produces a rapid response, which in turn activates adaptive immune responses, leading to acute ocular discomfort symptoms and/or visual dysfunction in the eyes. In the present disclosure, "chronic course of VDT-associated dry eye disease" refers to the long-term persistent stage of VDT-associated dry eye disease, which may be associated with dysfunction of ocular surface immune homeostasis, leading to sustained chronic inflammatory responses, accompanied by persistent symptoms such as eye dryness, irritation, and visual impairment. The intensity of these symptoms may fluctuate but does not completely resolve.

The technical solutions of the present disclosure will be further described in detail below with reference to specific embodiments. It should be understood that the following embodiments are merely illustrative and explanatory of the present disclosure and should not be construed as limiting the scope of protection of the present disclosure. All technologies implemented based on the above content of the present disclosure are encompassed within the scope intended to be protected by the present disclosure.

Unless otherwise stated, the raw materials and reagents used in the following examples are commercially available products or can be prepared by known methods.

### Example 1 Generation of Fibroblast-Like Epithelial Cells and Differential Gene Expression During Dry Eye Disease in Mice

### I. Experimental Materials

### 1. Animals

SPF-grade wild-type female C57BL/6J mice (age: 6-8 weeks; body weight: 18-25g) were obtained from Shanghai Jiesijie Experimental Animal Co., Ltd. Inclusion criteria for experimental mice: healthy mice with no corneal infection, corneal ulcer, corneal scar, and leukoplakia under slit-lamp examination, and with corneal fluorescein sodium staining score less than 10. All experimental procedures complied with the regulations of the Association for Research in Vision and Ophthalmology (ARVO) regarding the use of animals in ophthalmic and vision research, and the protocol was approved by the Institutional Animal Care and Use Committee of Wenzhou Medical University.

### 2. Main Instruments

| Instrument | Manufacturer |
|---|---|
| Adjustable industrial dehumidifier (Model ZD-890c) | Hangzhou Ouyi Limited Company |
| Intelligent humidity control mainframe (Model 13x) | Shanghai Hengye Limited Company |
| Microscopy imaging system (Model Imager.Z1) | Oberkochen Company, France |
| 10X Chromium Controller | 10x Genomics Company, USA |
| DNBSEQ platform | BGI Genomics Co., Ltd |
| Agilent 2100 Bioanalyzer | Agilent Technologies, USA |
| DNA EngniePeltier Thermal Cycler PCR system | BIO-RAD Laboratories, USA |
| ABI 7500 Quantitative PCR System | Applied Biosystems, USA |
| DU-800 UV-Vis Spectrophotometer | Beckman Coulter, Inc., USA |
| 5415R Refrigerated Microcentrifuge | Eppendorf SE, Germany |
| 5810R Refrigerated Centrifuge | Eppendorf SE, Germany |
| Attune NxT V6 Flow Cytometer | Thermo Fisher Scientific, USA |
| CO2 Incubator | Thermo Scientific, USA |
| Dissecting Microscope | Nikon, Japan |
| Micropipette | Eppendorf SE, Germany |
| Cell Counter | Shanghai Ruiyu Biotechnology |
| Ophthalmic Microsurgical Instrument | Suzhou 66Vision Co., Ltd. |

### 3. Primary Reagents

| Reagent | Supplier |
|---|---|
| MACS Tissue Storage Solution | Miltenyi Biotec, Germany |
| Collagenase IV | Gibco, USA |
| DMEM Medium | Gibco, USA |
| 0.4% Trypan Blue Stain | Thermo Scientific, USA |
| Red Blood Cell Lysis Buffer | Sigma-Aldrich, Germany |
| Dead Cell Removal Kit | Miltenyi Biotec, Germany |
| 10X Genomics Single Cell Second Generation | 10x Genomics Company, USA |
| Library Prep Kit | |
| Chromium Single Cell 3' Kit | 10x Genomics Company, USA |
| RNA Isolation Kit | Applied Biosystems, USA |
| M-MLV Reverse Transcription Kit | Applied Biosystems, USA |
| SYBR Green PCR Master Mix | Applied Biosystems, USA |
| Primer | Beijing Tsingke Biotech |
| Foxp3 Staining Buffer Kit | Thermo Scientific, USA |
| CD16/32 FcR block | Biolegend, USA |
| Anti-mouse Cytokeratin Pan Monoclonal | Invitrogen, USA |
| APC anti-mouse CD140a | Thermo Scientific, USA |
| Anti-mouse Aquaporin 3 | Abcam, UK |
| LYVE1 Antibody | Thermo Scientific, USA |
| BV421 Rat Anti-Mouse CD68 | Becton, Dickinson and Company |
| APC/Cy7 Anti-mouse CD45 | Becton, Dickinson and Company |
| CD72 Antibody | Thermo Scientific, USA |
| PE-Cy7 Hamster Anti-Mouse CD11c | Becton, Dickinson and Company |
| 0.1% PBS buffer, 1000 ml, powder | Fuzhou Maixin Biotech, Ltd |
| Fetal calf serum (FBS) | Gibco, USA |
| RPMI 1640 medium | HyClone, USA |
| EDTA buffer | Invitrogen, USA |

### 4. Antibodies and Reagents Used for Flow Cytometry

APC/Cy7 anti-mouse CD45 (Cat: 557659, clone: 30-F11, BD), Alexa Fluor 488 anti-mouse Cytokeratin Pan Monoclonal (Cat: MA5-18156, clone: C-11, Invitrogen), APC anti-mouse CD140a (Cat: 17-1401-81, clone:APA5, eBioscience), anti-mouse Aquaporin 3 (Cat: ab125219, abcam), Rabbit IgG PE-conjugated Antibody (Cat: F0110, R&D), Brilliant Violet 421 anti-mouse CD68 (Cat: 566388, clone: FA/11, BD), eFluor 660 anti-mouse LYVE1 (Cat: 50-0443-82, clone: ALY7, eBioscience), PE-Cy7 anti-mouse CD11c (Cat: 558079, clone: HL3, BD), FITC anti-mouse CD72 (Cat: MMCD72101, clone: 10.1.D2, Invitrogen), PE anti-mouse CCR2 (Cat: 568093, clone: Y15-488.rMAb, BD), Brilliant Violet 786 anti-mouse CD4 (Cat: 563727, clone: RM4-5, BD), Brilliant Violet 711 anti-mouse γδT (Cat: 563994, clone: GL3, BD), PerCP/Cyanine5.5 anti-mouse CD8a (Cat: 100734, clone: 53-6.7, BioLegend), Brilliant Violet 650 anti-mouse NK1.1 (Cat: 564143, clone: PK136, BD), Brilliant Violet 605 anti-mouse CD3 (Cat: 563004, clone: 145-2C11, BD). The dilution of fluorescently labeled antibodies was routinely 1:100.

### 5. PCR Primer Sequences

GAPDH: forward, 5'-ATGTTCGTCATGGGTGTGAA-3',
   reverse, 5'-GGTGCTAAGCAGTTGGTGGT-3';
Cdh1: forward, 5'-CAGGTCTCCTCATGGCTTTGC-3',
   reverse, 5'-CTTCCGAAAAGAAGGCTGTCC-3';
Cdh2: forward, 5'-AGCGCAGTCTTACCGAAGG-3',
   reverse, 5'-TCGCTGCTTTCATACTGAACTTT-3';
Snai1: forward, 5'-CACACGCTGCCTTGTGTCT-3',
   reverse, 5'-GGTCAGCAAAAGCACGGTT-3';
Zeb1: forward, 5'-GCTGGCAAGACAACGTGAAAG-3',
   reverse, 5'-GCCTCAGGATAAATGACGGC-3';
Twist1: forward, 5'-TCGCAAGAGACGCAGCAGTC-3',
   reverse, 5'-CCTCCGCCCGCAGATTTCTT-3'.

### 6. Antibodies Used for Immunofluorescence Staining

anti-E-Cadherin antibody (dilution: 1:200, Cat: 14-3249-82, Thermo), anti-N-cadherin antibody (dilution: 1:200, Cat: PA5-85341, Thermo), Alexa Fluor 488 cross-adsorbed secondary antibody, donkey anti-rabbit IgG (H+L) (dilution: 1:300, Cat: A-21206, Thermo), Alexa Fluor 594 cross-adsorbed secondary antibody, goat anti-rat IgG (H+L) (dilution: 1:300, Cat: A-11007, Thermo).

### II. Experimental Methods

### 1. Dry Eye Disease Animal Modeling

To induce the VDT-associated dry eye disease model, mice were placed in an Intelligent Controlled Environment System (ICES) simulating VDT environment (humidity 15±3%, wind speed 2 m/s, temperature 22±1°C) for 1 week and 3 weeks, designated as VDT-associated dry eye disease 1-week and 3-week groups. Control mice were fed in a standard environment (humidity 60%-80%, temperature 22±1°C) without any treatment. At weeks 0, 1, and 3, corneal epithelial staining was graded according to the standard grading system (National Eye Institute, Bethesda, MD, USA) in a single-blind manner to evaluate the condition of the corneal epithelium.

### 2. Preparation of Single-Cell Suspensions

After euthanizing the mice, the palpebral and bulbar conjunctiva were excised to obtain conjunctival tissue. Conjunctival tissue from three mice per group was pooled as one sample and stored in MACS Tissue Storage Solution (Miltenyi Biotec). The samples were washed in phosphate-buffered saline containing 20 mM EDTA and shaken at 37°C for 15 minutes. The tissues were then cut into fragments and digested in RPMI 1640 medium (Thermo Fisher Scientific) containing collagenase IV (Gibco; 1000 U/mL) at 37°C for 30 minutes, followed by filtration through a 70 µm cell strainer. Red blood cells and dead cells were removed using red blood cell lysis buffer (Sigma Aldrich) and Dead Cell Removal Kit (Miltenyi Biotec), respectively. 10 µL of the suspension was counted under an inverted microscope using a hemocytometer. Cells were observed under a microscope after trypan blue staining.

### 3. Library Preparation and Single-Cell Sequencing

Single-cell RNA sequencing libraries were generated using the Chromium Single Cell 3' Kit according to the manufacturer's instructions. The prepared single-cell suspension was processed using the Chromium Single Cell Controller instrument (10x Genomics, Pleasanton, CA, USA) to generate single-cell Gel Bead-In-Emulsions (GEMs). This was followed by reverse transcription, cDNA amplification, fragmentation, end repair, adapter ligation, library amplification, circularization, and generation of DNA Nanoballs (DNBs). The constructed libraries were sequenced with 100 bp paired-end reads on the BGI DNBseq platform (BGI, Shenzhen, China) using combinatorial Probe-Anchor Synthesis (cPAS).

### 4. Raw Data Processing and Quality Control

Cell Ranger (version 7.0.1, 10x Genomics) was used to map transcripts to the mouse reference genome (mm10-2020-A) and quantify reads, generating a gene expression matrix for each sample. The expression matrices were then imported into the Seurat R package (version 4.1.0) for preliminary quality control, with the following exclusion criteria: cells expressing fewer than 200 or more than 7,500 genes, cells with more than 80,000 detected molecules, and cells with mitochondrial gene proportion exceeding 15%. The Scrublet package in Python was used to identify potential doublets, with the expected doublet rate set to 0.13, and the threshold was adjusted using the scrub.call_doublets function based on the doublet score histogram of each sample. The quality-controlled datasets were normalized using Seurat's SCTransform, and Principal Component Analysis (PCA) was performed on the resulting 3,000 highly variable genes to obtain the top 50 principal components. These principal components were input into the Harmony package (version 0.1.0) to remove batch effects between different samples.

### 5. Clustering, Annotation, and Visualization

The optimal number of principal components (PCs) for clustering was determined using Seurat's ElbowPlot function. Unsupervised clustering was performed on the top 20 PCs at a resolution of 1.0 using the Shared Nearest Neighbor (SNN) algorithm. Clustering results were visualized using Uniform Manifold Approximation and Projection (UMAP). Marker genes for each clustering were identified using the FindMarkers function and compared with established cell type markers for cell annotation. In addition, specific strategies were adopted for each cell subpopulation dataset to remove clusters that did not meet the set criteria or overrepresented certain markers: for epithelial cells, the number of mitochondrial genes was corrected, and clusters with Cd3 expression exceeding 50% were removed. For immune cells, cell populations lacking Ptprc expression were excluded, and clusters with Lum or Pecam1 expression exceeding 50% were removed.

### 6. Single-Cell Sequencing - Enrichment Analysis and Gene Set Scoring

To characterize the biological processes of different cell subpopulations, ClusterProfiler package (version 3.18.1) was used to perform enrichment analysis of differentially expressed genes based on the Kyoto Encyclopedia of Genes and Genomes (KEGG) database, Gene Ontology (GO) database, and Hallmark database. Gene Set Variation Analysis (GSVA) was performed using the GSVA package (version 1.38.2), with the gsva function used to estimate pathway activity scores. Pathways with significant differences (adjusted p-value < 0.05) were visualized using the pheatmap package (v1.0.12) and ggplot2 package (v3.2.1) in R. Feature scores for gene sets were calculated using Seurat's AddModuleScore function, with gene sets downloaded from the Molecular Signatures Database (MSigDB).

### 7. Cell Trajectory and RNA Velocity Analysis

Monocle2 (version 2.26.0) was used to infer the cell developmental trajectory of epithelial cells. The gene expression matrix of all cells from these subpopulations was used as input for Monocle2. After quality control and selection of highly variable genes, the DDRTree algorithm was used for dimensionality reduction, and cell trajectories were visualized based on cell subpopulations and cell states. The velocyto package in python was used to generate loom files containing mRNA splicing and unsplicing information from the bam files obtained after alignment with Cell Ranger, which were then input into scVelo for RNA velocity calculation with the following parameters: counts=30, n_top_genes=2000, n_pcs=30, and n_neighbors=30.

### 8. Flow Cytometry

Single-cell suspensions were prepared as described above. For cell surface staining, cells were blocked with CD16/32 FcR-block (Cat: 101302, BioLegend) for 10 minutes. After staining with Fixable Viability Stain (Cat: 564406, BD), cells were stained with surface markers at 4°C for 30 minutes. For intracellular staining, cells were fixed and permeabilized with Foxp3/Transcription Factor Staining Buffer Set (Cat: 00-5523-00, Invitrogen), followed by intracellular staining with appropriate fluorescently labeled antibodies in permeabilization buffer at 4°C for 45 minutes. Cells were acquired on a flow cytometer (Attune Nxt V6, Thermo). Flow cytometry data were analyzed using Flowjo software (version 10.5.3, BD).

### 9. Real-Time Quantitative PCR

Total RNA was extracted from conjunctival tissue according to the manufacturer's instructions (Cat: 74106, RNeasy mini kit, Qiagen, Crawley, U.K.). cDNA was synthesized from 0.5 µg of total RNA using random primers and M-MLV reverse transcriptase (Cat: 28025013, Applied Biosystems, Paisley, UK). Quantitative real-time polymerase chain reaction (qRT-PCR) analysis was performed using Power SYBR Green PCR Master Mix (Cat: A25742, Applied Biosystems, Paisley, UK) and Applied Biosystems Quant Studio 6 Real-Time PCR System (Applied Biosystems, Paisley, UK). Results were analyzed using the comparative threshold cycle (CT) method and normalized with GAPDH as an endogenous reference.

### 10. Immunofluorescence Staining

Conjunctival tissue and eyeballs from three mice per group were embedded in Optimal Cutting Temperature compound (Cat: 4583, SAKURA) and frozen in liquid nitrogen. The samples were then sectioned into 10 µm-thick sections and stored at -80°C. For staining, sections were thawed at room temperature. After thawing, they were fixed in 4% formaldehyde for 15 minutes. Then, sections were blocked with blocking buffer (Cat: ab64226, Abcam) containing 0.4% Triton X-100 (Cat: T8787, Sigma Aldrich) at room temperature for >60 minutes. Subsequently, sections were incubated with primary antibodies and corresponding fluorescently conjugated secondary antibodies according to the manufacturer's recommended sequence. 4',6-Diamidino-2-phenylindole (Cat: S36938, DAPI, Invitrogen) was added for 5 minutes. Images were captured using a laser scanning confocal microscope (LSM880, Carl Zeiss Meditec, Sartrouville, Germany).

### III. Experimental Results

FIG. 1A, based on single-cell sequencing analysis, revealed that conjunctival epithelial cells were abnormally increased in VDT-associated dry eye disease, especially during the acute phase, including basal epithelial cells highly expressing basal epithelial cell markers KRT15, KRT6A, and KRT14; fibroblast-like epithelial cells (FL Epi) co-expressing basal epithelial cell markers and fibroblast marker CD140a; and endothelial-like epithelial cells (EL Epi) co-expressing basal epithelial cell markers and endothelial marker Pecam1. In addition, superficial epithelial cells (Sup Epi) and goblet cells were decreased. FIG. 1B shows that these increased epithelial cell subpopulations specifically expressed water channel protein AQP3, while this phenomenon was not observed in other epithelial cell subpopulations or stromal cells. FIG. 1C shows that the fibroblast-like epithelial subpopulation was detected using flow cytometry, confirming the existence of this subpopulation, and it was found that this subpopulation was significantly increased during VDT-associated dry eye disease while also highly expressed AQP3 (FIG. 1D). FIG. 1E shows that cell trajectory analysis revealed that the fibroblast-like epithelial subpopulation was derived from the proliferation and differentiation of basal epithelial cells. These results indicate that epithelial cells undergo proliferation and differentiation during VDT-associated dry eye disease and generate new cell subpopulations with fibroblast-like characteristics.

FIG. 2A shows that gene set scoring revealed that epithelial cell stemness was significantly upregulated at both 1 week and 3 weeks of VDT-associated dry eye disease, while the Epithelial-Mesenchymal Transition (EMT) pathway showed specific upregulation at 1 week of VDT-associated dry eye disease, with fibroblast-like epithelial cells obtaining the highest EMT score among epithelial cell subpopulations, suggesting that their generation may be related to the EMT process. FIG. 2B shows that EMT associated markers (CDH2, SNAI1, ZEB1, TWIST1) were significantly upregulated at 1 week of VDT-associated dry eye disease, with TWIST1 being the most prominent. Real-time quantitative PCR also revealed that Twist1, Snai1, and Zeb1 were significantly upregulated at 1 week of VDT-associated dry eye disease (FIG. 2C), with Twist1 being specifically upregulated in acute dry eye disease but showing no significant difference compared to the normal group in chronic dry eye disease (FIG. 2D). FIG. 2E shows gene set signature scoring of epithelial cell subpopulations revealed that the TGF-β signaling pathway, an important pathway for inducing EMT, was significantly upregulated in the fibroblast-like epithelial subpopulation at 1 week of VDT-associated dry eye disease. Compared to the control group, the conjunctival epithelium during VDT-associated dry eye disease showed a shift from E-cadherin to N-cadherin expression and migration toward the conjunctival stromal layer, displaying a classic epithelial-mesenchymal transition (EMT) phenomenon (FIG. 3). On the other hand, at 1 week of VDT-associated dry eye disease, fibroblast-like epithelial cells became the major source of chemokines among epithelial cells, with a slight downregulation at 3 weeks of VDT-associated dry eye disease (FIG. 2E). This may suggest that fibroblast-like epithelial cells mainly play an important role in recruiting immune cells and promoting inflammatory responses in VDT-associated dry eye disease.

From the above results, it can be seen that in the mouse ICES model simulating VDT-associated dry eye disease, conjunctival epithelial cells strongly express the specific marker AQP3 and undergo EMT phenomenon, with fibroblast-like epithelial cells obtaining the highest EMT score among epithelial cell subpopulations, indicating that their generation is associated with the EMT process. FIG. 3B and FIG. 4 show that EMT associated markers (CDH2, SNAI1, ZEB1, and TWIST1) were significantly upregulated at 1 week of VDT-associated dry eye disease and remained at high levels at 3 weeks, with TWIST1 and SNAI1 being the most prominent. The goblet cell marker MUC5AC showed significantly decreased expression during the acute phase of VDT-associated dry eye disease and partial recovery during the chronic phase.

### Example 2 Close Association of Fibroblast-Like Epithelial Cells with Inflammatory Responses

### I. Experimental Materials

The following antibodies and reagents were used for immunofluorescence staining: anti-CCL2 antibody (dilution: 1:100, Cat: 127-545-160, Thermo), anti-Aquaporin 3 antibody (dilution: 1:100, Cat: ab125219, Abcam), Alexa Fluor 488-conjugated secondary antibody, goat anti-Armenian Hamster IgG (H+L) (dilution: 1:300, Cat: 127-545-160, Jackson), Alexa Fluor 594-conjugated secondary antibody, goat anti-rabbit IgG (H+L) (dilution: 1:300, Cat: ab150080, Abcam).

### II. Experimental Methods

### 1. Single-Cell Sequencing - Cell Communication Analysis

Other single-cell sequencing analysis methods were the same as in Example 1.

In the present disclosure, the CellChat package (version 1.5.0) was used to infer cell-cell interaction networks between different cell types based on the ligand-receptor pairs from the CellChatDB mouse database. The number of communications between groups was compared, and results were visualized using the netVisual_bubble function. Important ligand-receptor pairs for specific pathways were extracted, and key cell-cell communications were visualized using the netVisual_individual function. The information flow of each signaling pathway was compared, and overall information flow was visualized using rankNet. The netVisual_aggregate function revealed the strength of specific signaling pathways, and network centrality scores and the netAnalysis_signalingRole_network function were used to identify major signaling roles. NicheNet analysis used Nichenetr (version 1.1.1) to predict ligand-target gene relationships. All epithelial cells were defined as signal-receiving cells, and other cell types as signal-sending cells, to predict potential ligands regulating epithelial cell differentiation during dry eye disease. The minimum LFC method was used to infer ligand-target gene links with a cutoff of 0.75, and upregulated receptor-ligand pairs in dry eye disease were visualized. Ligand-target gene heatmaps show the potential regulatory scores of top-ranked ligands and their target genes.

### 2. Flow Cytometry

Specific steps were as described in Example 1.

### 3. Immunofluorescence

Mouse conjunctiva and eyeballs were excised and embedded in OCT (optimal cutting temperature compound, Cat: 4583, SAKURA), frozen in liquid nitrogen, and then sectioned into 10 µm sections. Sections were stored at -80°C. For staining, sections were thawed at room temperature (RT). After thawing, sections were fixed in 4% formaldehyde for 15 minutes. Then, sections were blocked with blocking buffer (Cat: ab64226, serum-free protein blocking buffer, Abcam) supplemented with 0.4% Triton X-100 (Cat: T8787, Sigma Aldrich) at RT for 60 minutes. Sections were incubated with primary antibodies and corresponding fluorescently labeled secondary antibodies according to the manufacturer's recommended sequence. 4',6-Diamidino-2-phenylindole (Cat: S36938, DAPI, Invitrogen) was added for 5 minutes. Images were captured using a laser scanning confocal microscope (LSM880, Carl Zeiss Meditec, Sartrouville, Germany).

### III. Experimental Results

FIG. 5A shows through gene set variation analysis that fibroblast-like epithelial cells in VDT-associated dry eye disease were enriched in "IL-2/STAT-5 signaling pathway," "IL-6/JAK/STAT3 signaling pathway," and "inflammatory response." FIG. 5B also shows that this population had the highest acute inflammatory response score, which was at its highest value at 1 week. This means that this subpopulation plays an important role in promoting inflammatory responses during the acute phase of VDT-associated dry eye disease. FIG. 5C shows that MHC-II molecules were significantly upregulated in epithelial cells during VDT-associated dry eye disease, which would contribute to activating pathogenic T cells and initiating adaptive immune responses, while fibroblast-like epithelial cells showed extensive upregulation of chemokines such as CCL2, CCL7, and CCL8 at 1 week of VDT-associated dry eye disease (FIG. 5D), which can bind to CCR2 and mediate monocyte/macrophage recruitment. FIG. 5E shows cell communication analysis revealing a significant increase in interactions between epithelial cells and immune cells in VDT-associated dry eye disease. Systematic analysis of receptor-ligand interactions between epithelial cells and myeloid cells with CD4+ T cells revealed that fibroblast-like epithelial cells displayed the most abundant enhanced chemokine associated ligand-receptor pairs during dry eye disease, including Ccl2-Ccr2, Ccl7-Ccr2, Cxcl13-Cxcr3, and Cxcl10-Cxcr3 (FIG. 5F). Enhanced interactions between fibronectin Fn1 and integrins, which are common molecules involved in cell adhesion, were also observed. FIG. 5G detected enhanced communication of fibroblast-like epithelial cells through CCL2 expression with various immune cells (CD4+ T cells, γδT cells, ILC2, NK, and macrophages) at 1 week of VDT-associated dry eye disease, indicating their recruitment effect on these immune cells.

In FIG. 6A, immunofluorescence staining confirmed co-localization of CCL2 with epithelial cells highly expressing AQP3 and identified a population of basal epithelial cells with high CCL2 expression and stromal expansion in VDT-associated dry eye disease. In FIG. 6B-C, using flow cytometry, it was found that CCR2, the receptor for CCL2, was highly expressed in various immune cells on the ocular surface, with CD68+ macrophages and CD4+ T cells being the major cell types expressing CCR2.

In FIG. 6D, using NicheNet analysis to connect ligands and target genes to predict cell-cell interactions, it was found that signals from myeloid cells potentially promoted epithelial cell differentiation toward fibroblast-like cells. Specifically, at 1 week of VDT-associated dry eye disease, TGF-β1 produced by myeloid cells (mainly macrophages) had the potential to induce epithelial cells to express collagen fiber-encoding genes such as Col1a1, Col1a2, Col3a1, Postn and the like. In addition, the macrophage-specific marker APOE had the activity to induce IGFBP2 in epithelial cells. FIG. 6E using PCR also confirmed the upregulation of TGF-β1 and IGFBP2 expression on the ocular surface in VDT-associated dry eye disease. This suggests that macrophages may enhance the pro-inflammatory effects of epithelial cells by secreting TGF-β1 and APOE to alter the expression patterns of epithelial cells in VDT-associated dry eye disease, and regulate the fate of epithelial cells differentiating toward fibroblast-like cells. Furthermore, epithelial cells, as the major regulatory hub for inflammation and stress signals, chemoattract infiltrating CD4+ T cells and myeloid cell populations, thereby maintaining the vicious cycle of dry eye inflammation.

From the above results, it can be seen that epithelial cells undergo fate changes during VDT-associated dry eye disease, generating a fibroblast-like epithelial cell subpopulation that exerts pro-inflammatory effects through chemokine secretion during the acute phase, actively regulating the ocular surface immune microenvironment. Effector molecules associated with this process can serve as biomarkers for VDT-associated dry eye disease diagnosis, including TGF-β1, which regulates epithelial cell fate changes, its target IGFBP2, and the key chemokines secreted by epithelial cells, CCL2, CCL7, and CCL8.

### Example 3 Upregulation of Factors Related to Epithelial Cell Phenotype Changes in Dry Eye Disease Patients with Prolonged VDT Use

### I. Experimental Materials

### Human Samples

A total of 26 healthy subjects aged 18-50 years participated in the study. The healthy subjects were individuals with no history of ocular trauma or surgery (including refractive surgery), no ocular history other than refractive error, no recent contact lens use, no autoimmune diseases or recent use of immunosuppressants, not participating in other clinical trials, and no systemic contraindications. All subjects provided informed consent prior to participation. All experimental procedures were approved by the Research Ethics Office of Wenzhou Medical University (ID: 2022-132-K-101-01) and strictly followed the approved guidelines. The study enrolled a total of 26 healthy subjects according to the following inclusion and exclusion criteria.

Exclusion criteria were as follows: previous history of ocular trauma, ocular surgery (including refractive surgery); diagnosed with ocular diseases other than dry eye disease and refractive error; contact lens wear within 1 week before examination; concurrent confirmed autoimmune disease or use of immunosuppressants within 1 month; patients concurrently participating in any other clinical trials; subjects judged by the investigator to have systemic diseases that may make them unsuitable for participation in this study.

### II. Experimental Methods

### 1. Study Design

The study lasted for 4 weeks (28 days) and was divided into two phases. Phase 1 (Week 0): subjects were required to maintain daily VDT use time ≤ 3 hours; Phase 2 (Weeks 1, 2, and 3): subjects were required to maintain VDT use time > 8 hours. Subjects were required to complete 4 questionnaire collections and ocular examinations at the end of Weeks 0, 1, 2, and 3, and ocular surface cell samples were collected 3 times at the end of Weeks 0, 1, and 3.

### 2. Subject Questionnaire Completion

Subjects completed the Ocular Surface Disease Index (OSDI) questionnaire according to their own conditions. To ensure the accuracy and reliability of the questionnaire, researchers explained the questionnaire to the subjects before they filled it out. In the OSDI questionnaire, "not applicable" options indicated that the subject had not recently engaged in the activity or had not been in the environment, while "no" options indicated that the subject had recently engaged in the activity or had been in the environment but was asymptomatic.

### 3. Subject Ocular Examinations

Examinations were performed in the following order according to the principle of non-invasive first, then invasive: meibography, slit-lamp biomicroscope examination, and ocular surface cell sample collection. Except for ocular surface cell samples collected from the right eye, all other examinations were performed on both eyes.

### 4. Slit-Lamp Examination

### 4.1 Corneal Fluorescein Sodium Staining

A fluorescein sodium filter strip was moistened with one drop of tobramycin eye drops and gently applied to the lateral palpebral conjunctival surface of the lower eyelid. Subjects were instructed to rotate their eyes, and corneal epithelial staining was observed under cobalt blue light using a slit-lamp microscope. Corneal epithelial defects stained green were observed. Corneal fluorescein sodium staining scores were recorded for five corneal regions: superior, inferior, nasal, temporal, and central. Each region was scored 0-3 based on severity, with a maximum of 15 points. Specific scoring was as follows: 0 point, no punctate staining; 1 point, 1-30 punctate stains; 2 points, ≥30 punctate stains without confluence; 3 points, punctate staining with confluence or filament formation.

### 4.2 Conjunctival Lissamine Green Staining

A lissamine green filter strip was moistened with one drop of tobramycin eye drops and gently applied to the lateral palpebral conjunctival surface of the lower eyelid. Subjects were instructed to rotate their eyes, and conjunctival staining was observed under white light using a slit-lamp microscope. Lissamine green staining scores were recorded for two conjunctival regions: nasal and temporal. Each region was scored 0-3 based on severity, with a maximum of 6 points. Specific scoring was as follows: 0 point: 0-9 staining points; 1 point: 10-32 staining points; 2 points: 33-100 staining points; 3 points: ≥100 staining points.

### 4.3 Fluorescein Sodium Tear Break-Up Time

After fluorescein sodium staining of the ocular surface, subjects were instructed to blink 3 times and then open their eyes. A stopwatch was used to record the time from eye opening to the first appearance of a black spot in the tear film, which was recorded as the tear break-up time. The examination was performed 3 times, and the average value was taken as the measurement result.

### 5. Diagnosis of Dry Eye Disease

According to the diagnostic criteria for dry eye disease proposed in the 2017 consensus report "New Perspectives on Dry Eye Disease Definition and Diagnosis: A Consensus Report of the Asian Dry Eye Society (ADES)", subjective symptoms combined with tear break-up time were used as diagnostic indicators. In this study, OSDI score ≥ 13 combined with FBUT < 5 seconds was used as the diagnostic criteria for dry eye disease.

### 6. Ocular Surface Cell Sample Collection and Preservation

Ocular surface cell samples were collected from the right eye of all subjects. One drop of proparacaine hydrochloride eye drops was instilled into the inferior conjunctival sac, and subjects were instructed to rotate their eyes. Two swabs were used to gently swab the superior and inferior palpebral conjunctival surfaces, respectively, 3 times each. One swab was used to gently swab the superior, inferior, nasal, and temporal bulbar conjunctiva once each. The tips of the 3 swabs were broken off and placed into TRIzol (Thermo Fisher, 15596018) and stored at -80°C for bulk RNA sequencing.

### 7. Transcriptome Sequencing

Total RNA was extracted from samples using TRIzol according to the manufacturer's instructions. The Bioanalyzer 2100 and RNA 6000 Nano LabChip Kit (Agilent, CA, USA, 5067-1511) were used to assess RNA concentration and integrity. cDNA libraries were sequenced on the Illumina NovaSeq^{™} 6000 platform with 150 bp paired-end reads. Cutadapt (v1.9) was used to filter out low-quality reads, and FastQC (v0.11.9) was used to verify sequence quality. Reads were mapped to the human reference genome (UCSC hg38) using HISAT2 (v2.0.4). Mapped reads for each sample were assembled using StringTie (v1.3.4d), and transcriptomes of all samples were compared and merged using gffcompare (v0.9.8). StringTie was used to estimate the expression levels of all transcripts and generate count tables. Transcripts per million (TPM) were used to quantify transcript expression differences between samples.

DESeq2 R package (v1.20.0) was used for differential expression analysis between two different groups. Genes with P value < 0.05 and absolute fold change > 2 were considered differentially expressed genes. GSVA analysis was performed to assess pathway enrichment differences between groups.

### III. Experimental Results

To investigate the impact of VDT environmental stress on ocular surface parameters, a prospective study with a self-controlled before-after design was employed, enrolling 26 healthy subjects (flowchart shown in FIG. 7A). As shown in FIG. 7B-C, with increasing duration of VDT exposure, corneal staining scores increased and tear break-up time decreased, with week 3 remaining stable compared to week 2. According to the diagnostic criteria for dry eye disease proposed by the Asian Dry Eye Society in 2017, using subjective symptoms combined with tear break-up time less than 5 seconds as diagnostic criteria for dry eye disease, it was found that after 1 week of prolonged VDT use, the prevalence of VDT-associated dry eye disease increased, reaching a maximum of 32.7% at week 3.

FIG. 7D shows that EMT signature scores were elevated in VDT-associated dry eye disease, indicating that EMT also occurs on the human ocular surface. FIG. 7E and FIG. 8 show that the EMT associated markers TWIST1 and SNAI1 were significantly upregulated in conjunctival superficial cells during VDT-associated dry eye disease, and the regulators associated with the EMT process, TGF-β1 and IGFBP2, also showed an upward trend. In addition, the key marker expressed by fibroblast-like epithelial cells, AQP3, as well as the chemokines CCL2, CCL7, and CCL8, were upregulated during VDT-associated dry eye disease, while the goblet cell marker MUC5AC showed significantly decreased expression in acute VDT-associated dry eye disease and partial recovery in chronic VDT-associated dry eye disease. The expression changes of these biomarkers were consistent with the trends observed in VDT-associated dry eye disease mice, indicating that the characteristics of epithelial cells acquiring fibroblast-like and pro-inflammatory phenotypes during the course of VDT-associated dry eye disease are conserved across species, and biomarkers associated with this process can aid in the diagnosis and precise staging patients with VDT-associated dry eye disease.

### Example 4 Evaluation of the Direct Role of Inhibiting Epithelial-Mesenchymal Transition and Fibrosis in the Treatment of VDT-associated dry eye disease

### I. Experimental Materials

### 1. Animals

SPF-grade wild-type female C57BL/6J mice (age: 6-8 weeks; body weight: 18-25g) were obtained from the Zhejiang Animal Center. Inclusion criteria for experimental mice: healthy mice with no corneal infection, corneal ulcer, corneal scar and leukoplakia under slit-lamp examination, and with corneal fluorescein sodium staining score less than 10. All experimental procedures complied with the regulations of the Association for Research in Vision and Ophthalmology (ARVO) regarding the use of animals in ophthalmic and vision research, and the protocol was approved by the Institutional Animal Care and Use Committee of Wenzhou Medical University.

### 2. Main Reagents

1) Preparation of 0.5% Anti-Fibrotic Agent Solution:
   (1) 20 mg of anti-fibrotic agent was weighed;
   (2) the agent was completely dissolved in 40 µL DMSO;
   (3) normal saline was slowly added to a total volume of 4 mL; and
   (4) the solution was placed on a shaker overnight to ensure thorough mixing;
      final concentration: 0.5% (w/v).
2) Preparation of Control Vehicle Solution:
   (1) 40 µL DMSO was measured;
   (2) normal saline was added to a total volume of 4 mL; and
   (3) mixed gently.

### 3. Antibodies and Reagents Used for Flow Cytometry

BV711 anti-mouse F4/80 (Cat: 565612, clone: T45-2342, BD). Other antibodies and reagents were as described in Example 1.

### 4. PCR Primer Sequences

Twist1: forward, 5'-TCGCAAGAGACGCAGCAGTC-3';
   reverse, 5'-CCTCCGCCCGCAGATTTCTT-3';
IL-1β: forward, 5'-GCAACTGTTCCTGAACTCAACT-3',
   reverse, 5'-ATCTTTTGGGGTCCGTCAACT-3';
IL-17a: forward, 5'-AAAGCTCAGCGTGTCCAAAC-3',
   reverse, 5'-ACGTGGAACGGTTGAGGTAG-3';
IFNγ: forward, 5'-ATGAACGCTACACACTGCATC-3',
   reverse, 5'-CCATCCTTTTGCCAGTTCCTC-3';
TNFα: forward, 5'-AGGCACTCCCCCAAAAGATG-3',
   reverse, 5'-CCACTTGGTGGTTTGTGAGTG-3'.

### 5. Antibodies Used for Immunofluorescence Staining

anti-IGFBP2 antibody (dilution: 1:500, Cat: ab188200, abcam), anti-CD45 antibody (dilution: 1:200, Cat: MAB114, R&D Systems). Other antibodies were as described in Example 1.

### II. Experimental Methods

### 1. Study Design

This example used the Intelligent Controlled Environment System (ICES) to establish a mouse model of VDT-associated dry eye disease. Mice were placed in the ICES under adverse conditions simulating a VDT environment for 2 weeks to induce VDT-associated dry eye disease formation. After modeling was completed, mice continued to be raised under ICES conditions to maintain the VDT-associated dry eye disease state, more closely resembling real-world conditions. Subsequently, a 4-week drug eye drop treatment intervention was initiated in VDT-associated dry eye disease mice. The drug was 0.5% anti-fibrotic agent pirfenidone or tranilast, administered twice daily, one drop per eye, approximately 6 µL per drop.

Four experimental groups were established in this example:
(1) Dry eye disease model untreated group (Control, n=9, total 18 eyes);
(2) Dry eye disease + normal saline eye drop group (Saline, n=10, total 20 eyes);
(3) Dry eye disease + pirfenidone eye drop treatment group (Pirfenidone, n=10, total 20 eyes);
(4) Dry eye disease + tranilast eye drop treatment group (Tranilast, n=10, total 20 eyes).

### 2. Corneal Fluorescein Sodium Staining

0.5 µL of 5% fluorescein sodium solution was instilled into the conjunctival sac of mice using a micropipette. After 3 minutes, corneal epithelial staining was graded under a slit-lamp microscope with cobalt blue light. Scoring was recorded by a fixed examiner in a masked manner, according to the National Eye Institute standard grading system. The cornea was divided into five regions: central, superior, inferior, nasal, and temporal. Each region was scored 0-4 grade (0: no punctate staining; 1 grade: 1-5 punctate stains; 2 grade: 6-15 punctate stains; 3 grade: 16-30 punctate stains; 4 grade: 30 or more punctate stains). The sum of scores from all regions was the total corneal fluorescein sodium staining score.

### 3. Real-Time Quantitative PCR Detection

Specific steps were as described in Example 1.

### 4. Flow Cytometry

Specific steps were as described in Example 1.

### 5. Immunofluorescence

Specific steps were as described in Example 1.

### III. Experimental Results

To further evaluate the direct role of inhibiting epithelial-mesenchymal transition and fibrosis in the treatment of VDT-associated dry eye disease, the mice were intervened starting from the baseline of the VDT-associated dry eye disease model and multi-level analysis was performed before and after treatment. The experimental results are as follows:
Corneal Staining and Symptom Relief:
Mice in each group were subjected to corneal fluorescein sodium staining and observed under a slit-lamp microscope at different time points (FIG. 1A). The results showed that after 1 week of treatment with anti-fibrotic agents, the degree of corneal staining in VDT-associated dry eye disease model mice was already significantly improved (FIG. 1A-B), and this improvement trend was maintained after 4 weeks of continuous treatment. This suggests that inhibiting epithelial fibrosis has early and sustained efficacy in VDT-associated dry eye disease.

Changes in Expression of Inflammation and Epithelial-Mesenchymal Transition (EMT) associated

### Markers:

Real-time quantitative PCR was used to detect the expression levels of the EMT key transcription factor Twist1 and various pro-inflammatory factors (IL-1β, IL-17a, IFN-γ, and TNF-α) in conjunctival tissue. After treatment with pirfenidone and tranilast, Twist1 was significantly downregulated; tranilast treatment significantly downregulated the expression of IL-17a, IFN-γ, and TNF-α genes (FIG. 1C, n=4), indicating that inhibiting epithelial fibrotic-like transformation can effectively reduce inflammatory responses associated with VDT-associated dry eye disease.

Epithelial-Mesenchymal Transition and Immune Cell Infiltration Analysis:
Flow cytometry results showed that in the VDT-associated dry eye disease model, the proportion of cells highly expressing CK PAN (epithelial marker) and CD140a (fibroblast marker) increased, accompanied by elevated CCL2 expression levels; after treatment with anti-fibrotic agents, this fibroblast-like epithelial cell subpopulation and CCL2 expression were significantly reduced (FIG. 1D, n=3). In addition, the proportion of F4/80+ macrophages in the conjunctiva of VDT-associated dry eye disease mice was significantly reduced after treatment (FIG. 1E, n=3).

Immunofluorescence detection further supported the above findings (FIG. 2A-C, n=3).

Anti-CCL2 and IGFBP2 staining showed that the expression of these two molecules in conjunctival tissue was significantly downregulated after anti-fibrotic agent treatment (FIG. 2A), indicating that epithelial-mesenchymal transition and fibrosis-related inflammatory chemotactic signals were effectively inhibited.

Anti-N-cad and E-cad staining results showed that EMT-related changes in epithelial cells were alleviated after treatment (FIG. 2B).

Anti-CD45 staining showed that the degree of immune cell infiltration was significantly reduced (FIG. 2C), verifying that the inflammatory microenvironment of VDT-associated dry eye disease was effectively improved.

In summary, inhibiting the epithelial-mesenchymal transition and fibrosis process has significant effects on alleviating VDT-associated dry eye disease symptoms and reducing ocular surface damage. The use of anti-fibrotic agents such as pirfenidone and tranilast can significantly improve corneal damage in VDT-associated dry eye disease mice, reduce the expression of pro-inflammatory and pro-fibrotic key factors such as Twist1 and CCL2 in ocular surface epithelial cells, and reduce immune cell infiltration, thereby alleviating inflammatory responses and promoting ocular surface damage repair. This further demonstrates the core concept of the present disclosure: intervention targeting the epithelial-mesenchymal transition and fibrosis processes is an effective strategy for alleviating VDT-associated dry eye disease inflammation and improving ocular surface damage, providing potential intervention directions and prospects for clinical treatment of VDT-associated dry eye disease.

The above-described embodiments are merely illustrative of several implementations of the present disclosure, and the descriptions thereof are relatively specific and detailed, but should not be construed as limiting the scope of the patent disclosure. It should be noted that for those of ordinary skill in the art, several variations and improvements can be made without departing from the concept of the present disclosure, and these all fall within the scope of protection of the present disclosure. Therefore, the scope of protection of the patent of the present disclosure should be determined by the appended claims.

## Claims

1. Use of a gene as a biomarker in dry eye disease in the manufacture of a reagent or kit for diagnosing dry eye disease or monitoring efficacy of treatment for dry eye disease, wherein the gene is selected from one or more of *AQP3, SNAI1, TWIST1, CCL2, CCL7, CCL8, IGFBP2, TGF-β, and MUC5AC*;
preferably, the dry eye disease is a video display terminal associated dry eye disease, and the genes comprise *TWIST1, SNAI1,* and *IGFBP2*.

2. Use of an agent for detecting expression of a target gene in the manufacture of a reagent or kit for diagnosing dry eye disease or monitoring efficacy of treatment for dry eye disease, wherein the target gene comprises one or more of *AQP3, SNAI1, TWIST1, CCL2, CCL7, CCL8, IGFBP2, TGF-β,* and *MUC5AC*;
preferably, the dry eye disease is a video display terminal associated dry eye disease, and the genes comprise *TWIST1, SNAI1,* and *IGFBP2*.

3. A reagent or kit for diagnosing dry eye disease or monitoring efficacy of treatment for dry eye disease, wherein the reagent comprises an agent for detecting the expression level of a gene in a biological test sample from a subject, wherein the gene is at least one of *AQP3, SNAI1, TWIST1, CCL2, CCL7, CCL8, IGFBP2, TGF-β,* and *MUC5AC*; and the kit comprises the agent;
preferably, the dry eye disease is a video display terminal associated dry eye disease, and the genes comprise *TWIST1, SNAI1,* and *IGFBP2*.

4. The reagent or kit according to claim 3, wherein the biological test sample from the subject is obtained from conjunctival superficial cells of the ocular surface.

5. Use of a gene as a biomarker in dry eye disease in the manufacture of a reagent or kit for distinguishing between acute and chronic courses of dry eye disease in a patient, wherein the gene is selected from one or more of *AQP3, CCL2, CCL7, CCL8,* and *MUC5AC*;
preferably, the dry eye disease is a video display terminal associated dry eye disease, and the genes comprise *AQP3, CCL2,* and *MUC5AC*.

6. Use of an agent for detecting the expression of a target gene in the manufacture of a reagent or kit for distinguishing between acute and chronic courses of dry eye disease in a patient, wherein the target gene comprises one or more of *AQP3, CCL2, CCL7, CCL8,* and *MUC5AC*;
preferably, the dry eye disease is a video display terminal associated dry eye disease, and the genes comprise *AQP3, CCL2,* and *MUC5AC*.

7. A reagent or kit for distinguishing between acute and chronic courses of dry eye disease in a patient, wherein the reagent comprises an agent for detecting the expression level of a gene in a biological test sample from a subject, wherein the gene comprises at least one of *AQP3, CCL2, CCL7, CCL8,* and *MUC5AC*; the kit comprises the agent;
preferably, the dry eye disease is a video display terminal associated dry eye disease, and the genes comprise *AQP3, CCL2,* and *MUC5AC*.

8. The reagent or kit according to claim 7, wherein the biological test sample from the subject is obtained from conjunctival superficial cells of the ocular surface.

9. A system for diagnosing dry eye disease or monitoring efficacy of treatment for dry eye disease, wherein the system performs the following steps:
(1) detecting the expression level value of a corresponding biomarker in a biological test sample from a subject with the reagent or kit according to claim 3 or 4; and
(2) comparing the detected expression level value of the gene with the normal or reference expression level value of the biomarker;
preferably, the biological test sample is obtained from conjunctival superficial cells of the ocular surface.

10. The system for diagnosing dry eye disease according to claim 9, comprising a data input module, a data comparison module, and a conclusion output module, wherein
the data input module is configured to input the detected expression level value of the target gene in the biological test sample from the subject;
the data comparison module is configured for comparing the expression level value of the target gene in the biological test sample from the subject with a control value, wherein the control value is the expression level value of the target gene in a sample from a healthy subject;
the conclusion output module is configured for outputting a conclusion according to the following criteria: if the expression level value of the target gene in the biological test sample from the subject is greater than or less than the control value, the subject has, or is suspected of having, dry eye disease; "greater than or less than" specifically can be a statistically significant "greater than or less than"; wherein the target genes whose expression level values in the biological test sample from the subject are greater than the control value are *TWIST1, SNAI1,* and *IGFBP2*; and the target gene whose expression level value in the biological test sample from the subject is less than the control value is *MUC5AC*; preferably, the dry eye disease is a video display terminal associated dry eye disease.

11. Use of an anti-fibrotic agent in the manufacture of a medicament for treating dry eye disease; preferably, the dry eye disease is a video display terminal associated dry eye disease, and the anti-fibrotic agent is tranilast and a derivative thereof or pirfenidone and a derivative thereof .
